# EUROPEAN PATENT SPECIFICATION

(11) **EP 2 186 904 B1**
(45) Date of publication and mention of the grant of the patent: **18.07.2012**
(21) Application number: 08019901.1
(22) Date of filing: 14.11.2008
(51) Int. Cl.: C12P 19/44

(54) **Method for producing glucose derivatives**
Verfahren zur Herstellung von Glucosederivaten
Procédé de production de dérivés de glucose

(43) Date of publication of application: 19.05.2010
(73) Proprietor: Technische Universität Graz, 8010 Graz (AT)
(72) Inventor: Gödl, Christiane, 8010 Graz (AT); Sawangwan, Thornthan, 8043 Graz (AT); Nidetzky, Bernd, 8010 Graz (AT)
(74) Representative: Sonn & Partner Patentanwälte

(56) References cited:
- WO-A-2008/034158
- FARIA ET AL: "Design of new enzyme stabilizers inspired by glycosides of hyperthermophilic microorganisms" CARBOHYDRATE RESEARCH, vol. 343, September 2008 (2008-09), pages 3025-3033, XP025625790
- EMPADINHAS ET AL: "To be or not to be a compatible solute: Bioversatility of mannosylglycerate and glucosylglycerate" SYSTEMATIC AND APPLIED MICROBIOLOGY, vol. 31, August 2008 (2008-08), pages 159-168, XP022851457
- GOEDL ET AL: "Recombinant sucrose phosphorylase from Leuconostoc mesenteroides: Characterization, kinetic studies of transglycosylation, and application of immobilised enzyme for production of alpha-D-glucose 1-phosphate" JOURNAL OF BIOTECHNOLOGY, vol. 129, 2007, pages 77-86, XP008087469
- POSPÍSIL ET AL: "Glucosylglycerate is an osmotic solute and an extracellular metabolite produced by Streptomyces caelestis" FOLIA MICROBIOLOGIA (PRAGUE), vol. 52, 2007, pages 451-456, XP008105717
- HUNTER ET AL: "A compound representing the D-glycerate terminus of the methylglucose-containing polysaccharide of Mycobacterium smegmatis" BIOCHEMISTRY, vol. 18, 1979, pages 4458-4465, XP002525530
- GOEDL ET AL: "Ein effizienter biokatalytischer Herstellungsprozess für 2-O-(alpha-D-Glucopyranosyl)-sn-glycerin, einen natürlichen Osmolyt und feuchthaltenden Zusatzstoff" ANGEWANDTE CHEMIE, vol. 120, December 2008 (2008-12), pages 10240-10243, XP002524586

## Description

The present invention relates to methods for producing 2-0-(α-D-glucopyranosyl)-R-glycerate and 2-O-(α-D-glucopyranosyl)-S-glycerate.

Simple and complex carbohydrates govern a diverse range of cellular functions, including energy storage, cell-wall structure, cell-cell interaction and signalling, host-pathogen interactions and protein glycosylation. They also serve a function as osmolytes and small molecules of extreme lifestyles. Glycosyltransferases (GTs) are the enzymes responsible for the synthesis of glycosides in nature whereas, glycosylhydrolases (GHs) have been evolved to degrade them. Among GT and GH classes, the glycoside phosphorylases (GPs) are special in several respects. GPs catalyze the phosphorolysis of α- and β-D-glycosides, mainly glucosides (Glc-OR) including disaccharides and oligo- or polysaccharides of varying degree of polymerisation. Glucosyl transfer to phosphate (Pi) is favoured thermodynamically in vivo because phosphate is usually present in large excess over α-D-glucose 1-phosphate (Glc 1-P). However, thermodynamic equilibrium constants (K_{eq}) of GP-catalysed reactions are intermediate of K_{eq} values for the reaction of GTs (K_{eq} « 1) and GHs (K_{eq} » 1). The relatively favourable K_{eq} values and the fact that phospho-activated sugars are less expensive than nucleotide-activated ones, which are required by most GTs, make GPs interesting bio-catalysts for the stereo- and regiospecific synthesis of glucosides.

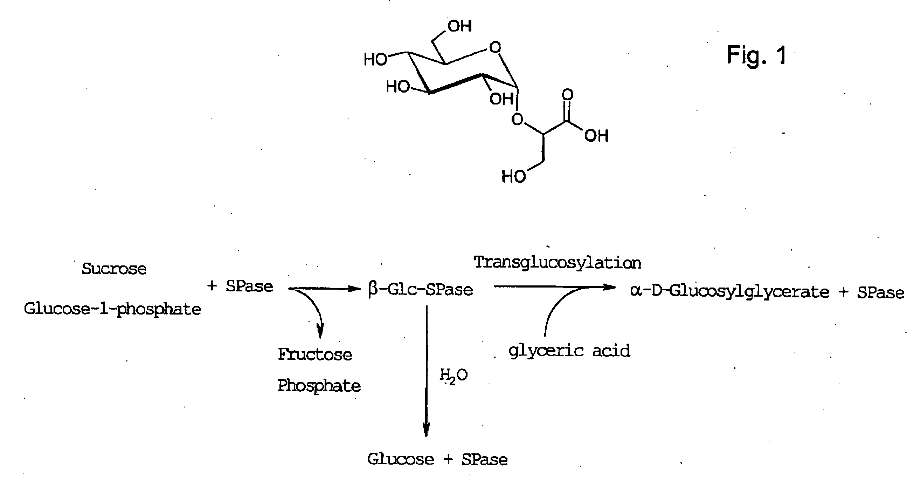

2-O-(α-D-glucopyranosyl)-R-glycerate (α-D-glycosyl glycerate; GGA, Fig. 1) is a naturally occurring substance which is synthesized by hyperthermophile and thermophile microorganisms. GGA is structurally similar to mannosylglycerate which is known in the art under the name Firroin. GGA can be isolated from nature in a very low amount and to date no chemical or biological process for producing GGA is known.

Faria et al. (Carbohydrate Res., 343 (2008): 3025-3033) discloses substances derived from hyperthermophilic microorganisms which can be used to stabilize enzymes.

The scientific article of Empadinhas et al. (Sys. Appl. Microbiol. 31 (2008): 159-168) relates to mannosyl and glucosyl glycerate.

WO 2008/034158 discloses a method for producing 2-0-glyceryl-α-D-glucopyranoside.

Hunter et al. (Biochem. 18 (1979): 4458-4465) relates to glucosyl glycerate.

It is an object of the present invention to provide an enzymatic method for the production of stereochemical pure 2-O-(α-D-glucopyranosyl)-R-glycerate and 2-O-(α-D-glucopyranosyl)-S-glycerate in high yield. Furthermore the method should preferably allow the use of economic substrates.

Therefore, the present invention relates to a method for producing 2-O-(α-D-glucopyranosyl)-glycerate from a glucosyl donor and a glucosyl acceptor comprising the steps:
- providing a sucrose phosphorylase (EC 2.4.1.7),
- incubating said sucrose phosphorylase with a mixture comprising a glucosyl donor and glyceric acid as glucosyl acceptor and
- isolating and/or purifying 2-O-(α-D-glucopyranosyl)-glycerate.

Sucrose phosphorylase (SPase; EC 2.4.1.7) catalyzes the conversion of sucrose and phosphate into D-fructose and Glc 1-P. SPase has been isolated from a number of bacterial sources. Genes encoding SPase have been cloned from different bacteria and expressed heterologously (Kawasaki H et al., Biosci. Biotech. Biochem. (1996) 60:322-324; Kitao S and Nakano E, J. Ferment. Bioeng. (1992) 73:179-184; van den Broek LAM et al., Appl. Microbiol. Biotechnol. (2004) 65:219-227). According to the systematic sequence-based classification of glycosylhydrolases (GH) and glycosyltransferases (GT) (Coutinho PM et al. J. Mol. Biol. (2003) 328:307-317; Henrissat B. Biochem. J. (1999) 280:309-316) SPase belongs to family GH13 (Clan GH-H), often referred to as the α-amylase family. The three-dimensional structure of SPase from Bifidobacterium adolescentis has been solved recently, revealing an (β/α)8 barrel fold and a catalytic site in which two carboxylate groups probably fulfill the role of a nucleophile (Asp192) and a general acid/base (Glu232).

The reaction of SPase proceeds with net retention of the anomeric configuration and occurs through a double displacement mechanism involving two configurationally inverting steps: cleavage of the carbon-oxygen bond of the glucosyl donor and formation of a covalent β-glucosyl-enzyme (β-Glc-E) intermediate; and reaction of the intermediate with phosphate to yield Glc 1-P. In a side reaction, the β-Glc-E intermediate may be intercepted by water, leading to hydrolysis. Hydrolytic conversion of sucrose is irreversible but proceeds nearly two orders of magnitude slower than the phosphorolytic reaction. SPase also catalyzes transglucosylation reactions which occur in competition with hydrolysis and whereby the β-Glc-E intermediate is attacked by external nucleophiles and new α-D-glucosides are produced.

Biochemical studies have shown that SPase is strictly specific for transferring a glucosyl moiety and does not tolerate structural modifications on the glucopyranosyl ring including epimerisation and deoxygenation. The list of known glucosyl donors for SPase includes - among others - sucrose, Glc 1-P, α-D-glucose 1-fluoride, etc... By contrast, the specificity of SPase for glucosyl acceptors is comparably relaxed.

It has been surprisingly found that SPase forms only 2-O-(α-D-glucopyranosyl)-R-glycerate (but also the S antipode) when incubated with a glucosyl donor and R-glyceric acid (or S-glyceric acid) as acceptor. This is rather surprising because theoretically naturally occurring R-glycerate may form 6 isoform glucosyl glycerates. Next to the selectivity of the glyceridic binding (α, β) also the regioselectivity of the glucosylation of glycerate is crucial. Therefore, it was surprising that SPase catalyses the transfer of the sugar moiety only to the secondary hydroxyl group of the glyceric acid.

The selectivity of SPase which forms only 2-O-(α-D-glucopyranosyl)-R-glycerate and 2-O-(α-D-glucopyranosyl)-S-glycerate in a high quantitative yield (> 95%) are crucial points of the method according to the present invention. The method of the present invention may use readily available substrates (which are both available from large-scale industrial processing) without any chemical derivatisation (required in chemical synthesis) and is characterised by an extremely high atom efficiency because all substrate converted goes quantitatively into product. In the method of the present invention only one enzyme is required and this may be of natural or recombinant preparation, used as free or immobilised, as isolated enzyme or in another catalyst form (permeabilized or resting cells).

The method of the present invention is preferably performed in vitro with purified enzyme or an enzyme extract (i.e. extract of recombinant or naturally occuring cells expressing SPase), whereby the SPase employed may be obtained from at least one source, which means that also SPases of more than one type (origin) may be employed.

Synthesis of 2-O-(α-D-glucopyranosyl)-R-glycerate and/or 2-O-(α-D-glucopyranosyl)-S-glycerate is preferably performed using a protein concentration of sucrose phosphorylase delivering an activity of between 1,000 and 1,000,000 units/litre (one unit is defined as the enzyme activity that converts 1 µmol of substrate per min under standard reaction conditions, typically 30 °C, reported in the literature.)

"Sucrose phosphorylase" as used herein refers to enzymes of the EC 2.4.1.7 class. Such molecules include also fusion proteins of sucrose phosphorylase with other peptides, polypeptides or proteins, which exhibit potentially also enzymatic or binding activities.

According to a preferred embodiment of the present invention the glucosyl donor is selected from the group consisting of sucrose and analogues of sucrose in which the fructosyl moiety has been modified or substituted by another ketosyl residue, Glc 1-P, α-D-glucose-1-fluoride, further stable, activated glucosyl donors such as α-D-glucose-1-azide, and mixtures thereof.

The glucosyl donor to be employed in the method of the present invention can be any one which serves as substrate for the transglycosylation reaction catalysed by the SPase.

The use of sucrose (a disaccharide consisting of glucose and fructose) in the method of the present invention will lead not only to the formation of 2-O-(α-D-glucopyranosyl)-R-glycerate or 2-O-(α-D-glucopyranosyl)-S-glycerate but also to the formation of fructose. If substrates like Glc 1-P or α-D-glucose-1-fluoride are employed, phosphate or fluoride will be products formed in addition to 2-O-(α-D-glucopyranosyl)-R-glycerate or 2-O-(α-D-glucopyranosyl)-S-glycerate. The achievable yield will depend on the energy content of the glucosyl donor and is greater than 30%, preferably greater than 40%, and in particular greater than 50%.

The glyceric acid used in the method of the present invention can be L- and/or D-gylceric acid or a racemate thereof, whereby 2-O-(α-D-glucopyranosyl)-R-glycerate is formed by using D-glyceric acid as glucose acceptor and 2-O-(α-D-glucopyranosyl)-S-glycerate using L-glyceric acid as glucose acceptor. The enantio-selectivity of the reaction is dependent on the substrate used [D-glyceric acid: 2-O-(α-D-glucopyranosyl)-R-glycerate; L-glyceric acid: 2-O-(α-D-glucopyranosyl)-S-glycerate].

The sucrose phosphorylase used in a method according to the present invention is preferably of microbial, preferably bacterial origin.

The advantage of using microbial SPases is the simple production, isolation and stability of these enzymes. They can be obtained from microorganisms naturally or recombinantly expressing SPase.

According to a preferred embodiment of the present invention the bacterial sucrose phosphorylase is obtained from Agrobacterium vitis (NCBI P33910), Bifidobacterium adolescentis (Q84HQ2), Bifidobacterium longum (Q84BY1), Escherichia coli (P76041), Escherichia coli 06 (Q8FHS2), Lactobacillus acidophilus (Q7WWP8, Q7WWQ5), Lactobacillus delbrueckii subsp. lactis (Q71I99), Leuconostoc mesenteroides (Q59495, Q9R5Q3), Listeria monocytogenes (Q4ENE7, Q4EQR2, Q4ETN7, Q4EHA0, Q4EJW2, Q4ELY7), Pseudomonas putrefaciens, Pseudomonas saccharophila (AAD40317), Rhodopirellula baltica (Q7UIS9), Shewanella baltica (Q3Q4P1), Shewanella frigidimarina (Q3NMD1), Solibacter usitatus (Q43TL5), Streptococcus mutans (P10249) and/or Synechococcus sp. (068858, Q7U3J7).

It is particularly preferred to use at least one SPase derived from Leuconostoc mesenteroides.

The SPase is preferably recombinantly produced as a full-length protein or a catalytically active fragment thereof or a fusion protein. However, it is of course also possible to use SPase directly from the organism which naturally produces said SPase. Methods for the recombinant production of SPase are known to the person skilled in the art (e.g. Sambrook J. et al. Molecular cloning: a laboratory manual. ISBN 0-87969-309-6).

As used herein, "full-length protein" refers to SPase encoded by a gene derived from an organism as, for instance, listed above. Said naturally occurring gene, in particular the SPase encoding region of said gene, is directly employed for the recombinant production of SPase.

"A catalytically active fragment" of SPase refers to protein fragments of SPase which have the same or substantially the same activity and substrate specificity as native SPase. The length of the fragments is not crucial provided that the fragments will have the same or similar substrate specificity and catalyse the formation of the same products as native SPase.

As used herein, "a fusion protein" refers to SPase or catalytically active fragments thereof recombinantly fused to at least one further protein, polypeptide or peptide. Said at least one further protein, polypeptide or peptide may be of any kind (e.g. enzyme).

It is noted that within the scope of the invention also variants (i.e. mutations including deletions, substitutions and insertions) of SPase are summarised, provided that these variants have the same or substantially the same (e.g. increased catalytical activity) activity as native SPase.

According to the present invention the SPase may be employed in the incubation step as either a cell-free enzyme, which may but need not be partially purified, a whole-cell system pretreated physically or chemically for improved permeability of the cell membrane (permeabilisation) and mechanical stability, encapsulated catalyst in which said free enzyme or whole-cell system are entrapped, preferably in gel-like structures, or immobilised on a carrier. A recent comprehensive summary of methods of enzyme immobilisation, including the permeabilisation of cells is given by Cao L., Carrier-bound Immobilised Enzymes (2005) Wiley-VCH, Weinheim.

Advantageously the SPase is immobilised on a carrier which preferably is a solid support. Any material that binds said SPase noncovalently, preferably natural or nonnatural polymers with anion exchange properties, or covalently, preferably a polymer, more preferably an acrylic polymer, in particular a copolymer of methacrylamide, N,N'-methylen-bis(acrylamide) and a monomer carrying oxirane groups.

The carrier is preferably a chromatography resin, preferably selected from the group consisting of anion exchange chromatography resin, cation exchange chromatography resin, affinity chromatography resin (e.g. comprising immobilised SPase specific antibodies) and hydrophobic interaction chromatography resin.

The SPase for use in the present invention may be immobilised (temporarily or covalently) on any carrier, preferably particles (e.g. beads), in particular chromatography resin, provided that the enzymatic activity of the enzyme is not affected in a way to change its substrate specificity or to reduce its activity to low conversion rates.

The carrier may comprise functional groups which require - in order to bind the SPase on the resin - that also the enzyme carries corresponding binding partners (e.g. streptavidin - biotin, chelated metal ions - His₆-tag).

To improve the affinity of the enzyme to carriers lacking said functional groups, SPase may be recombinantly produced as a fusion protein harbouring a binding peptide, preferably one showing ion-exchange properties, or a binding domain, preferably a polysaccharide binding domain, in particular a cellulose binding domain.

Several advantages are offered by the use of insoluble immobilised enzymes (carrier-bound, encapsulated, whole-cell systems) in the method of the present invention:
1. the immobilised enzyme is easily recovered from the reaction mixture at the conclusion of the reaction for reuse, whereas the soluble enzyme is only recovered with difficulty and loss of activity;
2. the immobilised enzyme is more stable than the soluble enzyme, both for the number of enzyme turnovers obtained versus the soluble enzyme, as well as for recovered enzyme activity at the conclusion of a reaction or after prolonged storage in aqueous buffer.

No specific method of immobilisation can be chosen for a particular enzyme with the expectation that the immobilisation will be successful. Furthermore, the expectation for successful co-immobilisation of more than one enzyme is even less predictable. It is generally agreed by those skilled in the art that a successful immobilisation of any enzyme must be discovered by screening a variety of methods, and an optimal result obtained by trial and error. The immobilisation of SPase on a carrier stabilizes the enzyme activity. Literature shows that entrapment of the enzyme also improves the stability (Soetaert W. et al., Progress in Biotechnology Vol. 10 (Petersen S.B., Svensson, B., Pederesen, S., Eds), Elsevier, Amsterdam). The immobilisation of enzymes can be performed using a variety of techniques, including: (1) binding of the enzyme to a carrier or support, via covalent attachment, physical adsorption, electrostatic binding, or affinity binding, (2) crosslinking with bifunctional or multifunctional reagents, (3) entrapment in gel matrices, polymers, emulsions, or some form of membrane, and (4) a combination of any of these methods. Detailed descriptions of many of these methods of enzyme immobilisation, and the various factors affecting the choice of a method of immobilisation, are collected in the following volumes of Methods in Enzymology, K. Mosbach (ed.), Academic Press, New York: Vol. 44 (1976), Vol. 135 (1987), Vol. 136 (1987), Vol. 137 (1988), and the references therein.

The immobilisation of SPase on oxirane acrylic beads Eupergit C and Eupergit C-250L (Rohm Pharma) resulted in a catalyst (enzyme + carrier) which was particularly stable to the reaction conditions and had a sufficiently high specific activity (units of enzyme activity/gram of catalyst). However, to be useful in the method of the present invention, both free and immobilised preparations of SPase can be used.

However, many of the deficiencies of the soluble enzymes can be eliminated by employing the immobilised enzyme catalyst. The stability of immobilised SPase in aqueous buffers is much greater than the soluble enzyme. Recovery and reuse of the immobilised catalyst was easily performed by simply filtering the catalyst away from the reaction mixture and recycling it to fresh reaction mixture; in this manner for immobilised SPase a high number of turnovers (i.e., the number of substrate molecules that are converted to product molecules per catalyst molecule before inactivation of the enzyme) can be achieved.

The immobilised SPase used in the reaction should be present in an effective concentration, usually a concentration of about 0.001 to about 100.0 IU/ml, preferably about 10 to about 50 IU/ml. An IU (International Unit) is defined as the amount of enzyme that will catalyze the transformation of one micromole of substrate per minute.

Upon completion of the reaction the SPase bound to a carrier may be removed by filtration or centrifugation. If the immobilised SPase is packed in a column (e.g. chromatographic column) the production of 2-O-(α-D-glucopyranosyl)-R-glycerate and 2-O-(α-D-glucopyranosyl)-S-glycerate can be achieved in a continuous way without the necessity of removing the immobilised SPase from the reaction mixture.

According to a preferred embodiment of the present invention the incubation of the SPase with the substrates is performed at a pH value of 4 to 10, preferably of 5 to 9, more preferably of 6 to 8, in particular of 7.

The pH value in the method according to the present invention is preferably selected from the ranges identified above, which allows an efficient conversion of the substrates into GGA.

According to another preferred embodiment of the present invention the incubation is performed for at least 15 min, preferably for at least 60 min, more preferably for at least 3 hours, even more preferably for at least 5 hours, more preferably for at least 12 hours, even more preferably for at least 72 hours.

The incubation of the substrates with the immobilised or unbound SPase may be performed for at least 15 minutes. However it is especially preferred to select the incubation time between 1 and 48 or between 5 and 24 hours. The incubation time depends also on the incubation temperature chosen. This means if the incubation temperature is below the optimal temperature of the enzyme the incubation time may be extended.

According to a preferred embodiment of the present invention the incubation is performed at a temperature range of 10 to 50°C, preferably of 15 to 40°C, more preferably at a temperature of 30°C.

The mixture which according to the present invention is incubated with the SPase comprises the glucosyl donor, in particular sucrose, in a concentration of 0.01 to 3 mol/l, preferably of 0.05 to 2 mol/l, more preferably of 0.1 to 1.5 mol/l, more preferably of 0.3 to 0.8 mol/l.

It turned out that the activity of the SPase and its substrate turnover leading to GGA is optimal in the glucosyl donor concentrations disclosed herein.

According to a preferred embodiment of the present invention the substrate mixture comprises glyceric acid in a concentration of 0.01 to 2 mol/l, preferably of 0.05 to 1 mol/l, more preferably of 0.1 to 0.8 mol/l, even more preferably of 0.3 to 0.6 mol/l.

The ratio of glyceric acid to glucosyl donor in the mixture ranges preferably from 0.1:1 to 10:1, preferably from 0.3:1 to 5:1, more preferably from 0.4:1 to 1:1.

The 2-O-(α-D-glucopyranosyl)-R-glycerate and 2-O-(α-D-glucopyranosyl)-S-glycerate obtainable by the method according to the present invention can be isolated by different chromatographic methods, preferably by elution chromatography using a two-step process where anion exchange chromatography on a suitable resin is followed by adsorption chromatography on activated charcoal combined with celite as a filter aid. The product mixture obtainable by the method according to the present invention is loaded on a column of said material equilibrated in water, and elution of bound 2-O-(α-D-glucopyranosyl)-R-glycerate and/or 2-O-(α-D-glucopyranosyl)-S-glycerate is achieved with 15% ethanol. Fractions containing 2-O-(α-D-glucapyranosyl)-R-glycerate and/or 2-O-(α-D-glucopyranosyl)-S-glycerate, also show residual glycerate and product resulting from cleavage of glucosyl donor. The 2-O-(α-D-glucopyranosyl)-R-glycerate and/or 2-O-(α-D-glucopyranosyl)-S-glycerate is obtained in a yield of greater 50%, preferably greater 60%, in particular greater 70%. The purity of the product after chromatography is greater 80%, preferably greater 90%, in particular greater 95%. Following concentration under reduced pressure, solid GGA is preferably obtained by drying, preferably by lyophilisation.

Charcoal may preferably be used as suspension or more preferably packed in a column (e.g. chromatographic column). The reaction mixture potentially comprising the enzyme or residual enzyme and substrate is contacted with the charcoal (e.g. applied on a charcoal column) and successively eluted. This eluate or even the reaction mixture itself can be (further) purified using an ion exchange resin, for instance.

According to another preferred embodiment of the present invention the sucrose phosphorylase is obtained from Leuconostoc mesenteroides (Q59495, Q9R5Q3) and used as free or preferably immobilised enzyme preparation, preferably immobilised on an acrylic polymer, in particular a copolymer of methacrylamide, N,N'-methylen-bis(acrylamide) and a polymer carrying oxirane groups, wherein the immobilised sucrose phosphorylase is incubated with sucrose as glucosyl donor.

In particular an SPase derived from Leuconostoc mesenteroides immobilised on polymer particles (or gels) carrying oxirane groups was exceptionally stable and well suited for continuous reactions, for example.

The 2-O-(α-D-glucopyranosyl)-R-glyoerate, which may be obtained by the method of the present invention, comprises the natural occurring 2-O-(α-D-glucopyranosyl)-R-glycerate (in high amounts because SPase is able to specifically catalyze the formation of said GGA without significant formation of by-products resulting from transglucosylation).

If, for instance, sucrose is used as glucosyl donor a product comprising natural GGA and fructose is obtained. If Glc 1-P is used as glucosyl donor a product comprising natural GGA and phosphate is obtained.

Therefore a product obtainable by the method of the present invention may further comprise fructose, preferably in an equimolar amount to GGA.

GGA is a naturally occurring molecule (a glycoside; a carbohydrate derivative) which serves the function of an osmoprotective substance and stabilizer in various microorganisms. The outstanding properties of GGA and also of 2-O-(α-D-glucopyranosyl)-S-glycerate are of substantial interest for technological application. Uses of GGA 2-O-(α-D-glucopyranosyl)-S-glycerate and derivatives thereof include but are not limited to the fields of medicine (cancer therapy), cosmetics (moisturizing and stabilizing additive to a range of products), and food products (antidiabetics). Furthermore these substances are a very efficient stabilizer of biomolecules (proteins, lipids) and microorganisms. Since to date no method was known to efficiently produce 2-O-(α-D-glucopyranosyl)-R-glycerate and 2-O-(α-D-glucopyranosyl)-S-glycerate most of these applications were not accessible.

According to a preferred embodiment of the present invention a cosmetic preparation comprising 2-O-(α-D-glucopyranosyl)-R-glycerate and/or 2-O-(α-D-glucopyranosyl)-S-glycerate can be obtained with the method of the present invention.

The cosmetic products are especially characterised by the fact that they only comprise natural GGA, which may be obtained by the method of the present invention.

According to a further preferred embodiment of the present invention a pharmaceutical preparation comprising 2-O-(α-D-glucopyranosyl)-R-glycerate and/or 2-O-(α-D-glucopyranosyl)-S-glycerate can be obtained with the method of the present invention.

According to a further preferred embodiment of the present invention a food supplement comprising 2-O-(α-D-glucopyranosyl)-R-glycerate and/or 2-O-(α-D-glucopyranosyl)-S-glycerate can be obtained with the method of the present invention.

Preferably, 2-O-(α-D-glucopyranosyl)-R-glycerate and/or 2-O-(α-D-glucopyranosyl)-S-glycerate obtained by the method according to the present invention is used as sweetener. A particular aspect is the use as sweetener of mixtures of 2-O-(α-D-glucopyranosyl)-R-glycerate and 2-O-(α-D-glucopyranosyl)-S-glycerate and fructose obtainable by the method of the present invention.

A further application relates to the use of 2-O-(α-/D-glucopyranosyl)-R-glycerate and/or 2-O-(α-D-glucopyranosyl)-S-glycerate obtained by a method of the present invention as a stabilizing additive to preparations of biomolecules during storage or processing, in particular during drying. In particular, 2-O-(α-D-glucopyranosyl)-R-glycerate and/or 2-0-(α-D-glucopyranosyl)-S-glycerate can serve as a stabilizer of living microorganisms, proteins and lipid-derived structures. It can stabilize protein preparations, for example without being restricted thereto, antibodies, antibody fragments, and enzymes, against denaturation and loss of biological activity.

2-O-(α-D-glucopyranosyl)-R-glycerate and/or 2-O-(α-D-glucopyranosyl)-S-glycerate obtained by the method of the present invention can be used as an additive that can facilitate protein folding, in particular that of recombinant proteins, under conditions in vitro as well as in vivo.

The present invention is further illustrated by the following figures and examples without being restricted thereto.

Fig. 1 shows the stereochemical structure of 2-O-(α-D-glucopyranosyl)-R-glycerate (GGA) and the proposed mechanism of conversion of sucrose into 2-O-(α-D-glucopyranosyl)-R-glycerate or 2-O-(α-D-glucopyranosyl)-S-glycerate using Leuconostoc mesenteriodes sucrose phosphorylase (SPase).

Table 1 shows NMR data for GGA produced and purified according to the method of the present invention and demonstrate unequivocally the chemical structure in Fig. 1.

### Determination of product structure by NMR

¹H- and ¹³C-NMR signals of GGA are assigned along with COSY, DQF-COSY, TOCSY, HSQC, HMBC spectra.

The GGA produced was of ≥ 95% purity. NMR shifts are given in Table 1. The structure of the product is that shown in Figure 1.

**Table 1: NMR shifts of GGA**

| **Position** | **¹H [ppm]** | **Mult.** | **Koppl. [Hz]** | **Int** | **¹³C [ppm]** |
|---|---|---|---|---|---|
| 1 | 4,872 | d | 3,8 | 1H | 97,38 |
| 2 | 3,410 | dd | 9,8/3,8 | 1H | 71,55 |
| 3 | 3,688 | dd | 9,8/9,8 | 1H | 73,16 |
| 4 | 3,287 | dd | 9,8/9,1 | 1H | 69,36 |
| 5 | 3,659 | ddd | 9,1/5,1/2,8 | 1H | 72,18 |
| 6a | 3,715 | dd | 12,5/2,8 | 1H | 60,44 |
| 6b | 3,620 | dd | 12,5/5,1 | 1H | |
| 1' | - | | | | 177,08 |
| 2' | 4,071 | dd | 6,2/3,0 | 1H | 78,96 |
| 3a' | 3,696 | dd | 12,0/6,2 | 1H | 62,03 |

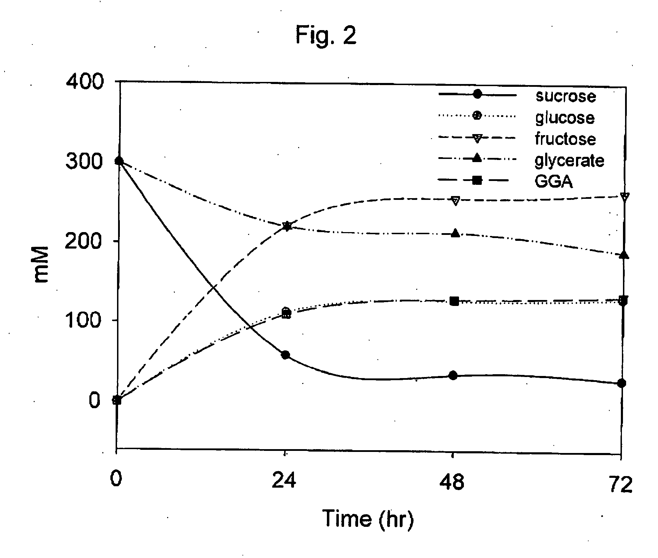

Fig. 2 shows the synthesis of GGA by enzymatic transglucosylation catalyzed by SPase. The reaction mixture, containing 0.3 mol/l sucrose as donor, 0.3 mol/l D-glyceric acid as acceptor and 20 U/ml SPase in 50 mM MES buffer (pH 7), was incubated at 30 ^{°}C and 550 rpm for 72 hr. Under these conditions, the analytical yield of GGA was 55%.

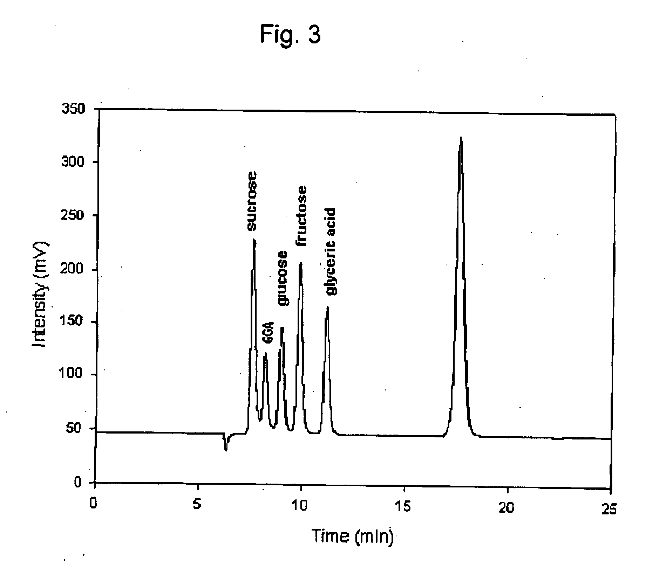

Fig. 3 shows the product analysed by HPLC. HPLC was performed using an Aminex HPX-87H column (300 x 7.8 mm) operated at a constant flow rate of 0.6 ml/min and 30 ^{°}C. 5 mM sulfuric acid (H₂SO₄) was applied as eluent. Refractive index detection was used. The graph shows an example of a reaction mixture, containing initially 0.3 mol/l sucrose as donor, 0.3 mol/l D-glyceric acid as acceptor and 20 U/ml SPase in 50 mM MES buffer (pH 7), after incubation at 30 °C and 550 rpm for 72 hr.

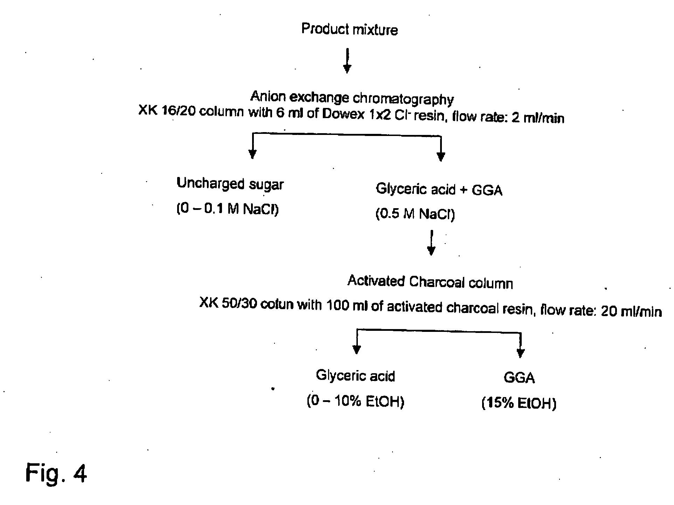

Fig. 4 shows the GGA purification protocol. The product mixture (which is composed of sucrose, glucose, fructose, D-glyceric acid, and GGA) was loaded onto a Dowex 1x2 Cl⁻ anion exchange chromatography column at a constant flow rate of 2 ml/min and 0.8 mPa. The elution was performed with a step gradient of 0.1 mol/l and 0.5 mol/l NaCl. The eluted fractions were analyzed by HPLC (HPX-87H, 5 mM H₂SO₄ as mobile phase at a flow rate of 0.6 ml/min and 30 °C). For the second chromatography step, an activated charcoal column was employed at a constant flow rate of 20 ml/min and 0.5 mPa. The elution was carried out with a step gradient of 10% and 15% of ethanol. Subsequently, the fractions were pooled and concentrated by evaporation and lyophilisation. Under these conditions, the yield of GGA was around 60%.

**Table 2:**

| **Sample** | **Sucrose** | **Glucose** | **Fructose** | **Glyceric acid** | **GGA** | **% yield of GGA** |
|---|---|---|---|---|---|---|
| | **[mg]** | **[mg]** | **[mg]** | **[mg]** | **[mg]** | |
| Product Mixture | 3.7 | 10.2 | 21.2 | 11.1 | 16.4 | 100 |
| Fraction after | | | | | | |
| Anion Exchange | | | | 6.4 | 11.6 | 70.9 |
| column | | | | | | |
| Fraction after | | | | | | |
| Activated Charcoal Column | | 0.1 | | 0.03 | 10.1 | 61.3 |

### EXAMPLES:

### Example 1: Production of SPase

SPase can be obtained as a native enzyme or as a recombinant enzyme (for example but not restricted thereto, produced in Escherichia coli) according to reports in literature (Guibert A and Monsan P, Ann. N.Y. Acad. Sci. (1988) 504:307-311; Vandamme EJ et al., Adv. Appl. Microbiol. (1987) 32:163-201; Kawasaki H et al., Biosci. Biotech. Biochem. (1996) 60:322-324; Kitao S and Nakano E, J. Ferment. Bioeng. (1992) 73:179-184; van den Broek LAM et al., Appl. Microbiol. Biotechnol. (2004) 65:219-227). It can of course be produced in various scales represented by shaken flask cultures of a suitable microorganism and bioreactors, preferably an aerated or nonaerated stirred tank reactor or a column reactor such as a bubble column or an airlift reactor. Partial purification or isolation of the enzyme is done using procedures described for SPase or by adopting general protocols of protein purification according to state of the art. Immobilisation procedures such as, for example but not restricted thereto, covalent and noncovalent binding to insoluble carriers, encapsulation, and whole-cell systems, is done using protocols already developed for SPase or by adopting general protocols according to state of the art (Pimentel MCB and Ferreira MSS, Appl. Biochem. Biotechnol. (1991) 27:37-43; Soetaert W. et al., Progress in Biotechnology Vol. 10 (Petersen S.B., Svensson, B., Pederesen, S., Eds), Elsevier, Amsterdam; Vandamme EJ et al., Adv. Appl. Microbiol. (1987) 32:163-201).

### Example 2: Enzyme assays

SPase activity was determined at 30°C using a continuous coupled enzymatic assay, in which production of Glc 1-P from sucrose and inorganic phosphate is coupled to the reduction of NAD⁺ in the presence of phosphoglucomutase (PGM) and glucose 6-phosphate dehydrogenase (G6P-DH). The standard assay was performed essentially as described elsewhere in 50 mM potassium phosphate buffer, pH 7.0, containing 10 mM EDTA, 10 mM MgCl₂ and 10 µM α-D-glucose 1,6-bisphosphate. The reaction mixture contained 250 mM sucrose, 2.5 mM NAD⁺, 3 U·ml⁻¹ PGM, 3.4 U·ml⁻¹ NAD⁺-dependent G6P-DH and the enzyme solution in appropriate dilution. The formation of NADH with time was monitored spectrophotometrically at 340 nm. One unit of SPase activity corresponds to the amount of enzyme that caused the reduction of 1 µmol of NRD⁺ per minute under the conditions described above. Protein concentrations were determined using the BioRad dye-binding method with bovine serum albumin as standard. Phosphate was determined colorimetrically at 850 nm and Glc 1-P was assayed in a coupled enzymatic system with PGM and G6P-DH.

### Example 3: Immobilisation of SPase onto a polymer containing oxirane groups

A total amount of 700 U of a preparation of crude SPase from Leuconostoc mesenteroides with a specific SPase activity of 50 U·mg⁻¹ was incubated at 4°C with 10 g of Eupergit C in 0.7 mol/l potassium phosphate buffer, pH 7.0, for 14 h. The agitation rate was 250 rpm. The immobilisate was washed several times with 20 mM MES buffer, pH 7.0. The binding efficiency, given by the ratio of the residual activity measured in the supernatant after the immobilisation and the total activity employed (U), was 0.5.

### Example 4: Operational stability of immobilised SPase

Eupergit C, onto which SPase was attached, was packed in a GE Healthcare XK26/40 glass column (2.6 cm; 53 ml or 111 ml, 34 U·g⁻¹ Eupergit C), equipped with a thermostatic jacket. The column was equilibrated with 20 mM MES buffer, pH 7.0. The substrate solution contained 600 mM of each, sucrose and phosphate in the same buffer, and was brought to reaction temperature (30°C) by incubation in a water bath. The solution was pumped through the packed bed at a constant flow rate of 6 ml·h⁻¹ delivered from a GE Healthcare piston pump (model P-500). The temperature at the outlet of the reactor was monitored continuously. At certain times, 1-ml samples were taken and used for further analysis.

The time course of Glc 1-P production in a continuous fixed bed reactor operated at a constant axial flow rate of 1.13 cm·h⁻¹ can be followed depending on the average residence time determined by the bed height, 8.8 h or 18.5 h, the conversion of sucrose (600 mM) was 68% and 91% respectively. The corresponding productivities, calculated as g·l⁻¹ product x reciprocal residence time, were 15.4 g·(l·h)⁻¹ and 10.9 g·(l·h)⁻¹. Note that the conversion rate remained constant up to extended reaction times of 650 h, emphasising the excellent stability of immobilised SPase under the operational conditions.

### Example 5: Synthesis of GGA using sucrose as donor

The reaction mixture, containing 0.3 mol/l sucrose as donor, 0.3 mol/l D-glyceric acid as acceptor and 20 U/ml Leuconostoc mesenteroides sucrose phosphorylase in 50 mM MES buffer (pH 7), was incubated at 30°C and 550 rpm for 72 hr. Under these conditions, the analytical yield of GGA (HPLC) was 55%. The analogous procedure employing L-glyceric acid is employed to synthesize the non-natural S or L enantiomer of GGA. The analytical yield was about 30%.

### Example 6: Purification of GGA

The product mixture (which is composed of sucrose, glucose, fructose, D-glyceric acid and glucosylglycerate; total mass ∼ 65 mg) was loaded onto a Dowex 1x2 Cl⁻ anion exchange chromatography column (6 ml volume; diameter 1.6 cm) at a constant flow rate of 2 ml/min and 0.8 mPa. The elution was performed with a step gradient of 0.1 mol/l and 0.5 mol/l NaCl. The eluted fractions were analyzed by HPLC (HPX-87H, 5 mM H₂SO₄ as mobile phase at a flow rate of 0.6 ml/min and 30°C). For the second chromatography step, an activated charcoal column (100 ml volume; diameter 5 cm) was employed at a constant flow rate of 20 ml/min and 0.5 mPa. The elution was carried out with a step gradient of 10% and 15% of ethanol. Subsequently, the fractions were pooled and concentrated by evaporation and lyophilization. Under these conditions, the yield of GGA was about 60%.

### Example 7: Optimization of GGA synthesis

The reaction mixture, containing 20 U/ml isolated enzyme, was incubated at 550 rpm for 72 hours. Unless otherwise mentioned glyceric acid was used in its D-form. Analytical yield is based on glucosyl donor used.

| **Sucrose [M]** | **Glyceric acid** | **Temperature** | **GGA** | **Yield** |
|---|---|---|---|---|
| | **[M]** | **[°C]** | **[mM]** | **[%]** |
| 0.3 | | | 166.3 | 55.5 |
| 0.4 | 0.3 | 30 | 190.6 | 47.7 |
| 0.5 | | | 235.1 | 47.0 |
| | 0.05 | | 62.1 | 20.7 |
| | 0.1 | | 97.4 | 32.5 |
| | 0.2 | | 123.5 | 41.2 |
| 0.3 | 0.3 | 30 | 175.8 | 58.6 |
| | 0.4 | | 178.5 | 59.5 |
| | 0.5 | | 171.3 | 57.1 |
| | 0.6 | | 172.5 | 57.5 |
| 0.3 | 0.3 | 25 | 176.0 | 58.7 |
| | | 30 | 177.5 | 59.2 |
| | | 35 | 174.7 | 58.2 |
| | | 40 | 169.8 | 56.6 |
| 0.3 | 0.5 | 25 | 156.4 | 52.1 |
| | | 30 | 171.8 | 57.3 |
| | | 35 | 151.7 | 50.6 |
| | | 40 | 156.6 | 52.2 |
| 0.3 | 0.3 L-form | 30 | 97.9 | 32.6 |
| | 0.3 DL-form | | 101.8 | 33.9 |
| 0.3 | 0.3 | 30 | 173.0 | 57.7 |
| 0.8 | | | 273.8 | 34.2 |

## Claims

1. Method for producing 2-O-(α-D-glucopyranosyl)-glycerate from a glucosyl donor and a glucosyl acceptor comprising the steps:
- providing a sucrose phosphorylase (EC 2.4.1.7),
- incubating said sucrose phosphorylase with a mixture comprising a glucosyl donor and glyceric acid as glucosyl acceptor and
- isolating and/or purifying 2-O-(α-D-glucopyranosyl)-glycerate.

2. Method according to claim 1, **characterised in that** the glucosyl donor is selected from the group consisting of sucrose and analogues of sucrose in which the fructosyl moiety has been modified or substituted by another ketosyl residue, α-D-glucose-1-phosphate, α-D-glucose-1-fluoride, further stable, activated glucosyl donors such as α-D-glucose-1-azide, and mixtures thereof.

3. Method according to claim 1 or 2, **characterised in that** the glyceric acid is L- and/or D-glyceric acid, whereby 2-O-(α-D-glucopyranosyl)-R-glycerate is formed using D-glyceric acid as glucose acceptor and 2-O-(α-D-glucopyranosyl)-S-glycerate using L-glyceric acid as glucose acceptor.

4. Method according to anyone of claims 1 to 3, **characterised in that** the sucrose phosphorylase is of microbial origin.

5. Method according to claim 4, **characterised in that** the bacterial sucrose phosphorylase is obtained from Agrobacterium vitis, Bifidobacterium adolescentis, Bifidobacterium longum, Escherichia coli, Escherichia coli 06, Lactobacillus acidophilus, Lactobacillus delbrueckii subsp. lactis, Leuconostoc mesenteroides, Listeria monocytogenes, Pseudomonas putrefaciens, Pseudomonas saccharophila, Rhodopirellula baltica, Shewanella baltica, Shewanella frigidimarina, Solibacter usitatus, Streptococcus mutans and/or Synechococcus sp..

6. Method according to any one of claims 1 to 5, **characterised in that** the sucrose phosphorylase is recombinantly produced as full-length protein or a catalytically active fragment thereof, or a fusion protein.

7. Method according to any one of claims 1 to 6, **characterised in that** the sucrose phosphorylase is prior the incubation step immobilised on a carrier.

8. Method according to any one of claims 1 to 7, **characterised in that** the mixture comprises the glucosyl donor in a concentration of 0.01 to 3 mol/l.

9. Method according to any one of claims 1 to 8, **characterised in that** the mixture comprises glyceric acid in a concentration of 0.01 to 2 mol/l.

10. Method according to any one of claims 1 to 9, **characterised in that** the ratio of glyceric acid to glucosyl donor in the mixture ranges from 0.1:1 to 10:1.

11. Method according to any one of claims 1 to 10, **characterised in that** the 2-O-(α-D-glucopyranosyl)-glycerate is isolated by elution chromatography on an anion exchange resin and on activated charcoal in a yield of 55%.

12. Method according to any one of claims 1 to 11, **characterised in that** the sucrose phosphorylase is obtained from Leuconostoc mesenteroides and immobilised on an acrylic polymer, in particular a copolymer of methacrylamide, N,N'-methylen-bis(acrylamide) and a polymer carrying oxirane groups, wherein the immobilised sucrose phosphorylase is incubated with sucrose as glucosyl donor.

13. Method according to any one of claims 3 to 12, **characterised in that** a cosmetic preparation comprising 2-O-(α-D-glucopyranosyl)-R-glycerate and/or 2-O-(α-D-glucopyranosyl)-S-glycerate is obtained.

14. Method according to any one of claims 3 to 12, **characterised in that** a pharmaceutical preparation comprising 2-O-(α-D-glucopyranosyl)-R-glycerate and/or 2-O-(α-D-glucopyranosyl)-S-glycerate is obtained.

15. Method according to any one of claims 1 to 12, **characterised in that** a food supplement comprising 2-O-(α-D-glucopyranosyl)-R-glycerate and/or 2-O-(α-D-glucopyranosyl)-S-glycerate is obtained.

16. Method according to any one of claims 3 to 12, **characterised in that** a stabilizer of biomolecules, in particular proteins or protein fragments and lipid-derived structures during processing and storage comprising 2-O-(α-D-glucopyranosyl)-R-glycerate and/or 2-O-(α-D-glucopyranosyl)-S-glycerate is obtained.

## Patentansprüche

1. Verfahren zur Herstellung von 2-0-(α-D-Glucopyranosyl)-glycerat aus einem Glucosyldonor und einem Glucosylakzcptor, umfassend die folgenden Schritte:
- das Bereitstellen einer Saccharosephosphorylase (EC 2.4.1.7),
- das Inkubieren der Saccharosephosphorylase mit einem Gemisch, das einen Glucosyldonor und Glycerinsäure als Glucosylakzeptor umfasst, und
- die Isolation und/oder Aufreinigung des 2-O-(α-D-Glucopyranosyl)-glycerats.

2. Verfahren nach Anspruch 1, **dadurch gekennzeichnet, dass** der Glucosyldonor ausgewählt ist aus der Gruppe bestehend aus Saccharose und Analoga von Saccharose, in denen die Fructosyleinheit durch einen weiteren Ketosylrest, α-D-Glucose-1-phosphat, α-D-Glucose-1-fluorid oder weitere stabile, aktivierte Glucosyldonoren so wie α-D-Glucose-1-azid modifiziert oder substituiert worden ist, sowie aus Gemischen daraus.

3. Verfahren nach Anspruch 1 oder 2, **dadurch gekennzeichnet, dass** es sich bei der Glycerinsäure um L- und/oder D-Glycerinsäure handelt, wobei jeweils das 2-0-(α-D-Glucopyranosyl)-R-glycerat unter Verwendung der D-Glycerinsäure als Glucoseakzeptor und das 2-0-(α-D-Glucopyranosyl)-S-glycerat unter Verwendung von L-Glycerinsäure als Glucoseakzeptor gebildet wird.

4. Verfahren nach einem der Ansprüche 1 bis 3, **dadurch gekennzeichnet, dass** die Saccharosephosphorylase mikrobiellen Ursprungs ist.

5. Verfahren nach Anspruch 4, **dadurch gekennzeichnet, dass** die bakterielle Saccharosephosphorylase aus Agrobacterium vitis, Bifidobacterium adolescentis, Bifidcbacterium longum, Escherichia coli, Escherichia coli 06, Lactobacillus acidophilus, Lactobacillus delbrueckii Subsp. lactis, Leuconostoc mesenteroides, Listeria monocytogenes, Pseudomonas putrefaciens, Pseudomonas saccharophi-1a, Rhodopirellula baltica, Shewanella baltica, Shewanella frigidimarina, Solibacter usitatus, Streptococcus mutans und/oder Synechococcus sp. erhalten wird.

6. Verfahren nach einem der Ansprüche 1 bis 5, **dadurch gekennzeichnet, dass** die Saccharosephosphorylase rekombinant in Form eines vollständigen Proteins oder eines katalytisch aktiven Fragments davon oder in Form eines Fusionsproteins hergestellt ist.

7. Verfahren nach einem der Ansprüche 1 bis 6, **dadurch gekennzeichnet, dass** die Saccharoscphosphorylase vor dem Inkubationsschritt auf einem Träger immobilisiert wird.

8. Verfahren nach einem der Ansprüche 1 bis 7, **dadurch gekennzeichnet, dass** das Gemisch den Glucosyldonor in einer Konzentration von zwischen 0,01 und 3 mol/l umfasst.

9. Verfahren nach einem der Ansprüche 1 bis 8, **dadurch gekennzeichnet, dass** das Gemisch die Glycerinsäure in einer Konzentration von zwischen 0,01 und 2 mol/l umfasst.

10. Verfahren nach einem der Ansprüche 1 bis 9, **dadurch gekennzeichnet, dass** das Verhältnis der Glycerinsäure zu dem Glucosyldonor in dem Gemisch in einem Bereich von zwischen 0,1:1 bis 10:1 liegt.

11. Verfahren nach einem der Ansprüche 1 bis 10, **dadurch gekennzeichnet, dass** das 2-0-(α-D-Glucopyranosyl)-glycerat unter Anwendung von Elutionschromatographie auf einem Anionenaustauschharz sowie auf Aktivkohle mit einem Ertrag von 55% isoliert wird.

12. Verfahren nach einem der Ansprüche 1 bis 11, **dadurch gekennzeichnet, dass** die Saccharosephosphorylase aus Leuconostoc mesenteroides erhalten und auf einem Acrylpolymer immobilisiert wird, insbesondere auf einem Copolymer von Methacrylamid, N, N'-Methylen-bis(acrylamid) und einem Polymer, das Oxirangruppen trägt, wobei die Inkubation der immobilisierten Saccharosephosphorylase mit Saccharose als Glucosyldonor erfolgt.

13. Verfahren nach einem der Ansprüche 3 bis 12, **dadurch gekennzeichnet, dass** eine kosmetische Zubereitung erhalten wird, die 2-0-(α-D-Glucopyranosyl)-R-glycerat und/oder 2-0-(α-D-Glucopyranosyl)-S-glycerat umfasst.

14. Verfahren nach einem der Ansprüche 3 bis 12, **dadurch gekennzeichnet, dass** eine pharmazeutische Zubereitung erhalten wird, die 2-0-(α-D-Glucopyranosyl)-R-glycerat und/oder 2-0-(α-D-Glucopyranosyl)-S-glycerat umfasst.

15. Verfahren nach einem der Ansprüche 1 bis 12, **dadurch gekennzeichnet, dass** ein Nahrungsmittelzusatz erhalten wird, der 2-0-(α-D-Glucopyranosyl)-R-glycerat und/oder 2-0-(α-D-Glucopyranosyl)-S-glycerat umfasst.

16. Verfahren nach einem der Ansprüche 3 bis 12, **dadurch gekennzeichnet, dass** im Verlauf der Verarbeitung und Lagerung ein Stabilisator von Biomolekülen, insbesondere von Proteinen oder Proteinfragmenten und von Lipiden abgeleiteten Strukturen, erhalten wird, der 2-0-(α-D-Glucopyranosyl)-R-glycerat und/oder 2-0-(α-D-Glucopyranosyl)-S-glycerat umfasst.

## Revendications

1. Procédé pour produire du 2-0-(α-D-glucopyranosyl)-glycérate à partir d'un donneur de glucosyle et d'un accepteur de glucosyle comprenant les étapes:
- fournir une saccharose phosphorylase (EC 2.4.1.7),
- incuber ladite saccharose phosphorylase avec un mélange comprenant un donneur de glucosyle et l'acide glycérique comme accepteur de glucosyle et
- isoler et/ou purifier le 2-O-(α-D-glucopyranosyl)-glycérate.

2. Procédé selon la revendication 1, **caractérisé en ce que** le donneur de glucosyle est choisi dans le groupe consistant en le saccharose et les analogues du saccharose où le groupement fructosyle a été modifié ou substitué par un autre résidu cétosyle, l'α-D-glucose-1-phosphate, l'α-D-glucose-1-fluorure, d'autres donneurs de glucosyle activés stables comme l'α-D-glucose-1-azide, et leurs mélanges.

3. Procédé selon la revendication 1 ou 2, **caractérisé en ce que** l'acide glycérique est l'acide L- et/ou D-glycérique, de sorte que le 2-O-(α-D-glucopyranosyl)-R-glycérate est formé avec l'acide D-glycérique comme accepteur de glucose et le 2-O-(α-D-glucopyranosyl)-S-glycérate avec l'acide L-glycérique comme accepteur de glucose.

4. Procédé selon l'une quelconque des revendications 1 à 3, **caractérisé en ce que** la saccharose phosphorylase est d'origine microbienne.

5. Procédé selon la revendication 4, **caractérisé en ce que** la saccharose phosphorylase bactérienne est obtenue à partir de Agrobacterium vitis, Bifidobacterium adolescentis, Bifidobacterium longum, Escherichia coli, Escherichia coli 06, Lactobacillus acidophilus, Lactobacillus delbrueckii subsp. lactis, Leuconostoc mesenteroides, Listeria monocytogenes, Pseudomonas putrefaciens, Pseudomonas saccharophila, Rhodopirellula baltica, Shewanella baltica, Shewanella frigidimarina, Solibacter usitatus, Streptococcus mutans et/ou Synechococcus sp..

6. Procédé selon l'une quelconque des revendications 1 à 5, **caractérisé en ce que** la saccharose phosphorylase est produite par recombinaison sous forme d'une protéine de longueur totale ou d'un fragment catalytiquement actif de celle-ci, ou d'une protéine de fusion.

7. Procédé selon l'une quelconque des revendications 1 à 6, **caractérisé en ce que** la saccharose phosphorylase est immobilisée sur un support avant l'étape d'incubation.

8. Procédé selon l'une quelconque des revendications 1 à 7, **caractérisé en ce que** le mélange comprend le donneur de glucosyle en une concentration de 0,01 à 3 mol/l.

9. Procédé selon l'une quelconque des revendications 1 à 8, **caractérisé en ce que** le mélange comprend l'acide glycérique en une concentration de 0,01 à 2 mol/l.

10. Procédé selon l'une quelconque des revendications 1 à 9, **caractérisé en ce que** le rapport de l'acide glycérique au donneur de glucosyle dans le mélange va de 0,1:1 à 10:1.

11. Procédé selon l'une quelconque des revendications 1 à 10, **caractérisé en ce que** le 2-O-(α-D-glucopyranosyl)-glycérate est isolé par chromatographie d'élution sur une résine échangeuse d'anions et sur charbon activé avec un rendement de 55%.

12. Procédé selon l'une quelconque des revendications 1 à 11, **caractérisé en ce que** la saccharose phosphorylase est obtenue à partir de Leuconostoc mesenteroides et immobilisée sur un polymère acrylique, en particulier un copolymère de méthacrylamide, N,N'-méthylène-bis(acrylamide) et un polymère portant des groupes oxirane, où la saccharose phosphorylase immobilisée est incubée avec le saccharose comme donneur de glucosyle.

13. Procédé selon l'une quelconque des revendications 3 à 12, **caractérisé en ce qu'**une préparation cosmétique comprenant du 2-O-(α-D-glucopyranosyl)-R-glycérate et/ou du 2-O-(α-D-glucopyranosyl)-S-glycérate est obtenue.

14. Procédé selon l'une quelconque des revendications 3 à 12, **caractérisé en ce qu'**une préparation pharmaceutique comprenant du 2-0-(α-D-glucopyranosyl)-R-glycérate et/ou du 2-O-(α-D-glucopyranosyl)-S-glycérate est obtenue.

15. Procédé selon l'une quelconque des revendications 1 à 12, **caractérisé en ce qu'**un complément alimentaire comprenant du 2-O-(α-D-glucopyranosyl)-R-glycérate et/ou du 2-O-(α-D-glucopyranosyl)-S-glycérate est obtenu.

16. Procédé selon l'une quelconque des revendications 3 à 12, **caractérisé en ce qu'**un stabilisant de biomolécules, en particulier de protéines ou de fragments de protéines et de structures dérivées de lipides pendant le traitement et le stockage comprenant du 2-0-(a-D-glucopyranosyl)-R-glycérate et/ou du 2-O-(α-D-glucopyranosyl)-S-glycérate est obtenu.
